# EUROPÄISCHE PATENTSCHRIFT

(11) **EP 2 129 649 B1**
(45) Veröffentlichungstag und Bekanntmachung des Hinweises auf die Patenterteilung: **27.12.2017**
(21) Anmeldenummer: 08717909.9
(22) Anmeldetag: 17.03.2008
(51) Int. Cl.: C07C 51/42, C07C 51/47, C07C 51/50, C07C 57/04, C07C 45/78, C07C 45/79, C07C 45/86, C07C 47/22, C07C 67/48, C07C 67/56, C07C 67/62, C07C 59/54

(54) **VERFAHREN DES TRANSPORTS EINER AUS EINEM LAGERBEHÄLTER ENTNOMMENEN FLÜSSIGEN MONOMERENPHASE IM TANK EINES TANKWAGENS ODER EINES TANKSCHIFFS**
METHOD FOR TRANSPORTING A LIQUID MONOMER PHASE, REMOVED FROM A STORAGE CONTAINER, IN THE TANK OF A TANKER LORRY OR TANKER SHIP
PROCEDE DE TRANSPORT D'UNE PHASE MONOMERE LIQUIDE PRELEVEE DANS UN CONTENANT DE STOCKAGE, DANS LA CITERNE D'UN CAMION-CITERNE OU D'UN NAVIRE-CITERNE

(30) Priorität: 23.03.2007 DE 102007014603; 23.03.2007 US 896582 P
(43) Veröffentlichungstag der Anmeldung: 09.12.2009
(73) Patentinhaber: BASF SE, 67056 Ludwigshafen am Rhein (DE)
(72) Erfinder: HEILEK, Jörg, 69245 Bammental (DE); HAMMON, Ulrich, 68163 Mannheim (DE); SCHLIEPHAKE, Volker, 67105 Schifferstadt (DE); MÜLLER-ENGEL, Klaus Joachim, 76297 Stutensee (DE); BLUM, Till, 67346 Speyer (DE)
(86) Internationale Anmeldenummer: PCT/EP2008/053174
(87) Internationale Veröffentlichungsnummer: WO 2008/116777

(56) Entgegenhaltungen:
- EP-A- 1 361 203
- DE-A1- 10 219 089
- US-A1- 2002 008 064

## Beschreibung

Vorliegende Erfindung betrifft ein Verfahren zum Befüllen des Tanks eines Tankwagens oder eines Tankschiffs mit einem Lagerbehälter zu entnehmender flüssiger Mononmerenphase oder des Transports einer aus einem Lagerbehälter entnommenen flüssigen Monomerenphase, im Tank eines Tankwagens oder eines Tankschiffs, wobei der Gehalt der flüssigen Monomerenphase an dem Monomeren ≥ 90 Gew.-% beträgt und das Monomere ein Monomeres aus der Gruppe bestehend aus Acrolein, Methacrolein, Acrylsäure, Methacrylsäure, Ester aus Acrylsäure und einem 1 bis 12 C-Atome aufweisenden Alkohol sowie Ester aus Methacrylsäure und einem 1 bis 12 C-Atome aufweisenden Alkohol ist.

Gemäß den vorstehenden Ausführungen beinhaltet der Begriff Monomer(e) in dieser Schrift Acrolein, Methacrolein, Acrylsäure, Methacrylsäure, Ester aus Acrylsäure und einem 1 bis 12 C-Atome aufweisenden Alkohol sowie Ester aus Methacrylsäure und einem 1 bis 12 C-Atome aufweisenden Alkohol. Als Alkohole kommen dabei sowohl einwertige (weisen eine -OH-Gruppe auf) als auch mehrwertige (weisen mehr als eine -OH-Gruppe auf) Alkohole in Betracht. Zu diesen Alkoholen gehören 1 bis 12, vorzugsweise 1 bis 8 C-Atome aufweisende ein- und mehrwertige Alkanole.

Diese Definition beinhaltet nicht in notwendiger Weise, dass die Herstellung der vorgenannten Ester durch Reaktion der entsprechenden Alkohole mit der jeweiligen Säure erfolgen muss. Vielmehr kommen als Herstellverfahren auch andere Reaktionen wie z. B. Umesterungen oder Additionsreaktionen in Betracht.

Als beispielhafte Monomere seien genannt Methylacrylat, Ethylacrylat, n-Butylacrylat, iso-Butylacrylat, tert.-Butylacrylat, 2-Ethylhexylacrylat, Hydroxyethylacrylat, Hydroxypropylacrylat, Hydroxymethylmethacrylat, Hydroxypropylmethacrylat, 2-Propylheptylacrylat, Methylmethacrylat, Ethylmethacrylat, n-Butylmethacrylat und tert-Butylmethacrylat.

Üblicherweise werden Monomere durch chemische Synthese erzeugt.

Dabei werden sie jedoch nicht unmittelbar in reiner Form befindlich, sondern als Bestandteil von gasförmigen oder flüssigen Produktgemischen erhalten, aus denen sie abgetrennt werden müssen. Diese Abtrennung wird in der Regel unter Anwendung thermischer Trennverfahren wie z.B. Absorption, Desorption, fraktionierende Kondensation, Extraktion, Kristallisation, Adsorption usw., bzw. unter Anwendung von Kombinationen verschiedener solcher thermischer Trennverfahren vorgenommen (vgl. z.B. DE-A 19 924 533, WO 2004/035 514, EP-A 1 388 533, EP-A 778 225, EP-A 1 041 062, EP-A 982 287, EP-A 982 288, WO 01/96 271, DE-A 10 336 386, DE-A 19 631 645, DE-A 19 501 325, EP-A 982 289, DE-A 19 838 845, WO 02/076 917, EP-A 1 695 954, EP-A 695 736, EP-A 778 225, EP-A 1 041 062, US 2004/0 242 826, EP-A 792 867, EP-A 784 046, EP-A 695 736, EP-A 112 5912, EP-A 1 097 741, WO 00/45 928, DE-A 2 246 480, DE-A 2 362 373, US-A 5,637,222 und der in diesen Schriften zitierte Stand der Technik).

Als Zwischenprodukt oder als Reinprodukt wird in der Regel eine flüssige Monomerenphase erhalten, deren Gehalt an dem jeweiligen Monomeren ≥ 90 Gew.-% oder ≥ 95 Gew.-% beträgt.

Häufig besteht der letzte Schritt auf dem Weg vom Produktgemisch zum Zwischen- oder Reinprodukt aus einer Kondensation aus einer gasförmigen Phase heraus oder aus dem Aufschmelzen einer kristallinen Phase. Beispielsweise kann die Kondensation in einer Rektifikationskolonne oberhalb der Zufuhrstelle des (in der Rektifikationskolonne) aufzutrennenden Gemischs in die Rektifikationskolonne aus den in der Rektifikationskolonne aufsteigenden Dämpfen heraus erfolgen und das Reinprodukt oberhalb des vorgenannten Zulaufs aus der Rektifikationskolonne entnommen werden. Dabei kann das aufzutrennende Gemisch in die Rektifikationskolonne sowohl flüssig (Rektifikation) als auch gasförmig (fraktionierende Kondensation) zugeführt werden.

In beiden Fällen, d.h., sowohl dann, wenn die flüssige Monomerenphase durch Kondensation aus einer gasförmigen Phase heraus, als auch dann, wenn die flüssige Monomerenphase durch Aufschmelzen einer kristallinen Phase (von Kristallisat) erzeugt wird, fällt sie normalerweise so an, dass sie bei visueller Betrachtung (d. h., bei Betrachtung mit bloßem menschlichem Auge) frei von Feststoffen ist. Dabei enthält die flüssige Monomerenphase üblicherweise radikalische Polymerisationsinhibitoren (Inhibitoren zur Unterdrückung unerwünschter radikalischer Polymerisation) gelöst, die regelmäßig im Verlauf ihrer Erzeugung und/oder als Abschluss einer solchen Erzeugung zudosiert werden. Im Fall einer Erzeugung durch Aufschmelzen einer kristallinen Phase kann das Polymerisationsinhibitorsystem aber auch bereits in der kristallinen Phase enthalten sein.

Grundsätzlich kann eine flüssige Monomerenphase als Zwischen- oder Reinprodukt mit einem Gehalt des jeweiligen Monomeren von ≥ 90 Gew.-% oder ≥ 95 Gew.-% einer Trennkolonne (z. B. einer Rektifikationskolonne) aber auch unterhalb der Zufuhrstelle des in einer Trennkolonnen (z. B. in einer Rektifikationskolonne) aufzutrennenden Gemischs in die Trennkolonne (z. B. in die Rektifikationskolonne) entnommen werden. Beispielsweise kann eine solche Entnahme auch aus dem Sumpfbereich der Trennkolonne (z. B. einer Rektifikationskolonne) heraus erfolgen. Dies wird z. B. dann der Fall sein, wenn man das Monomere (z. B. Acrylsäure) aus einem Produktgasgemisch seiner Herstellung heraus (z. B. Acrylsäure aus dem Produktgasgemisch einer heterogen katalysierten partiellen Gasphasenoxidation eines C₃-Vorläufers der Acrylsäure (z. B. Propylen, Propan, Glycerin, Acrolein) mit einer unter Normalbedingungen (25 °C, 1 bar) leichter als das Monomere (z. B. Acrylsäure) siedenden Flüssigkeit (im Fall von Acrylsäure z. B. Wasser, oder wässrige Lösungen) absorbiert und aus dem Absorbat nachträglich die leichter flüchtige Absorptionsflüssigkeit (gegebenenfalls mit Hilfe eines azeotropen Schleppmittels) rektifikativ vom Absorbat abtrennt (wobei häufig eine wie Eingangs beschriebene flüssige Monomerenphase verbleibt).

Die wie beschrieben erhältlichen flüssigen Monomerenphasen werden als Zwischen- oder als Reinprodukt normalerweise einem Lagerbehälter zugeführt. Enthält die flüssige Monomerenphase herstellungsbedingt ungelöstes, visuell sichtbares Polymerisat (die erfindungsgemäß relevanten Monomeren neigen bekanntermaßen alle in unerwünschter Wiese zu radikalischer Polymerisation; diese kann z. B. durch Licht, Temperatur und/oder Verunreinigungen ausgelöst werden und ist auch durch den Zusatz von Inhibitoren, die derartige radikalische Polymerisationen in gewissem Umfang zu unterdrücken vermögen, nicht völlig vermeidbar; die Herstellung der flüssigen Monomerenphasen erfolgt normalerweise im Beisein derartiger Polymerisationsinhibitoren, weshalb die flüssigen Monomerenphasen solche in der Regel herstellungsbedingt zugesetzt enthalten), so wird dieses regelmäßig abgetrennt, bevor die flüssige Monomerenphase in den Lagerbehälter überführt wird. In einfacher Weise kann eine solche Abtrennung von ungelösten Feststoffen durch Filtration mit Hilfe von Filtern entsprechender Maschenweite vorgenommen werden.

Üblicherweise werden solche Lagerbehälter auch als Tank bzw. auch als Lagertank bezeichnet. Das von einer fluiden Phase einnehmbare Innenvolumen eines Lagertanks (der in typischer Weise ruht) beträgt in der Regel 100 m³ bis 10000 m³, häufig 200 m³ bis 1000 m³ und charakteristisch 500 m³ (vgl. z. B. Research Disclosure Database Number 513001, published in January 2007). Zum Zweck der Einthaltung der erwünschten Lagertemperatur weist ein Lagertank in der Regel eine Vorrichtung auf, mit Hilfe derer (z. B. kontinuierlich) eine Teilmenge der gelagerten flüssigen Monomerenphase entnommen, über einen Wärmeaustauscher geführt und anschließend in den Lagertank rückgeführt wird bzw. rückgeführt werden kann (das erfindungsgemäße Verfahren kann nun auch so durchgeführt werden, dass die erfindungsgemäße Trennoperation bei der vorgenannten Entnahme und/oder Rückführung (z. B. durch in die Leitungen entsprechend eingebaute Filter (z. B. kontinuierlich) durchgeführt wird).

Da die vorgenannten Polymerisationsinhibitorsysteme ihre volle Wirkung normalerweise nur im Beisein von molekularem Sauerstoff (der seinerseits selbst Inhibitor ist) entfalten, werden die flüssigen Monomerenphasen üblicherweise unter einer molekularen Sauerstoff enthaltenden Gasatmosphäre gelagert (vgl. z.B. WO 2005/049 543 sowie US-A 6,910,511). D.h., das von einer fluiden Phase einnehmbare Innenvolumen des Lagertanks wird von der im Lagertank gelagerten flüssigen Monomerenphase nur teilweise ausgefüllt und das dabei verbleibende restliche einnehmbare Innenvolumen des Lagerbehälters von einer molekularen Sauerstoff enthaltenden Gasphase eingenommen. Mit Hilfe von Mischvorrichtungen wird in der Regel dafür Sorge getragen, dass die flüssige Monomerenphase an dem in ihr gelösten molekularen Sauerstoff nicht verarmt.

Im Lagertank (im Lagerbehälter) verbleibt die flüssige Monomerenphase so lange, bis von ihr zu Zwecken anderweitiger Verwendung entnommen wird. Eine solche Verwendung besteht unter anderem im Transport der dem Lagertank entnommenen flüssigen Monomerenphase zu einem Verbraucher.

In der Regel erfolgt dieser Transport im Tank eines Tankwagens (als Tankmaterial wird häufig Edelstahl verwendet). D. h., der Tank wird normalerweise von einem Lastkraftwagen (auf der Straße) oder einem Schienenfahrzeug (auf Schienen) transportiert. Das Fassungsvermögen (das von einem fluiden Medium einnehmbare Innenvolumen) des Tanks eines solchen Tankwagens beträgt in der Regel wenistens 5 m³, häufig wenigstens 10 m³ und vielfach 15 bis 40 m³ bzw. 20 bis 30 m³; teilweise ist sein Innenraum in wenigstens zwei, in der Regel wenigstens 3 oder wenigstens 4 gegeneinander völlig abgetrennte Kammern aufgeteilt, die voneinander unabhängig mit flüssiger Monomerenphase befüllt werden. Das Füllvolumen mit flüssiger Monomerenphase beträgt typisch ca. 80 bis 90 Vol.-% des von einer fluiden Phase jeweils einnehmbaren Innenvolumens. Die Tankbefüllung erfolgt normalerweise an Luft.

Häufig erfolgt der Transport aber auch auf dem Seeweg mit Hilfe eines Tankschiffs (im Tank desselben). Das Fassungsvermögen eines solchen Tankschiffs geht über dasjenige des Tanks eines Tankwagens normalerweise weit hinaus. In der Regel ist auch der Tankinnenraum eines Tankschiffs in voneinander abgetrennte Kammern aufgeteilt.

Wesentlich ist nun, dass ein solcher Transport von flüssiger Monomerenphase sicher erfolgt. Dazu gehört im besonderen, dass während des Transports der flüssigen Monomerenphase eine unerwünscht radikalische Polymerisation derselben weitestgehend vermieden wird. Dies nicht zuletzt deshalb, weil eine radikalische Polymerisation exotherm verläuft, was insbesondere bei ungekühltem Transport zu gegebenenfalls kritischer Eigenerwärmung der transportierten Monomerenphase führen kann (dies vor allem bei längeren Transportstrecken wie z. B. Überseetransporten).

Gleichzeitig ist aber auch ein möglichst geringer Gehalt der transportierten flüssigen Monomerenphase an radikalischem Polymerisationsinhibitor wünschenswert.

Dies ist zum einen darin begründet, dass es sich bei Polymerisationsinhibitoren um vergleichsweise kostspielige Wirkstoffe handelt. Zum anderen vermögen die Monomeren selbst und/oder deren spätere Reaktionsprodukte häufig mit den Inhibitoren unter Ausbildung von farbintensiven Reaktionsprodukten zu reagieren, was in der Regel unerwünscht ist. Weiterhin wirken sich die Polymerisationsinhibitoren bei einer Verwendung der gelagerten flüssigen Monomerenphase in radikalischen Polymerisationsreaktionen normalerweise nachteilig aus (z. B. auf die Produktqualität).

Weiterhin ist ein nicht zu hoher Sauerstoffgehalt der über der flüssigen Phase im Transporttank befindlichen Gasphase wünschenswert. Dies ist darauf zurückzuführen, dass Mischungen aus gasförmigem Monomer (dieses verdampft in natürlicherweise) und molekularem Sauerstoff explosiv sein können (vgl. WO 2004/007405, DE-A 10 2004 034 515, WO 2005/049543, Research Disclosure Database Number513001, published in January 2007 sowie Research Disclosure, Database Number 513002, published in January 2007). Insgesamt wird daher eine Minimierung des Sauerstoffgehalts im Transporttank angestrebt. Besonders vorteilhaft liegt sie unter der sogenannten Sauerstoffgrenzkonzentration (vgl. WO 2004/007405), unterhalb derer ein explosives Verhalten des Gasgemischs nicht möglich ist.

Die US 2002/0008064 A1 beschreibt eine Vorrichtung zum Entfernen von Feststoffen durch Filtration. Die DE 102 19089 A1 beschreibt ein Verfahren zum Transport und/oder Lagerung von Acrylsäure. Die EP 1 361 203 A2 betrifft ein Verfahren zur Herstellung reiner Methacrylsäure und die EP 1 810 959 A1 ein Verfahren zur Herstellung von Acrylsäure.

Vor dem vorgenannten Hintergrund bestand die Aufgabe der vorliegenden Erfindung insbesondere darin, ein Verfahren des Transports einer aus einem Lagerbehälter entnommenen flüssigen Monomerenphase, deren Gehalt an dem Monomeren ≥ 90 Ges.-% beträgt, im Tank eines Tankwagens oder eines Tankschiffs zur Verfügung zu stellen, das einen Transport mit erhöhter Sicherheit sowie gegebenenfalls verringertem Inhibitorbedarf ermöglicht.

Demgemäß wurde ein Verfahren zum Befüllen des Tanks eines Tankwagens oder eines Tankschiffs mit einem Lagerbehälter zu entnehmender flüssiger Monomerenphase oder des Transports einer aus einem Lagerbehälter entnommenen flüssigen Monomerenphaseim Tank eines Tankwagens oder eines Tankschiffs, wobei der Gehalt der flüssigen Monomerenphase and dem Monomeren ≥ 90 Gew.-% beträgt, und das Monomere ein Monomeres aus der Gruppe bestehend aus Acrolein, Methacrolein, Acrylsäure, Methacrylsäure, Ester aus Acrylsäure und einem 1 bis 12 C-Atome (vorzugsweise 1 bis 8 und besonders bevorzugt 1 bis 4 C-Atome) aufweisenden Alkohol sowie Ester aus Methacrylsäure und einem 1 bis 12 C-Atome (vorzugsweise 1 bis 8 und besonders bevorzugt 1 bis 4 C-Atome) aufweisenden Alkohol ist, gefunden, das dadurch gekennzeichnet ist, dass die flüssige Monomerenphase auf dem Weg aus dem Lagerbehälter in den Tank des Tankwagens oder des Tankschiffs wenigstens einer Trennoperation zur Abtrennung wenigstens einer Teilmenge (vorzugsweise wenigstens 25 Gew.-%, besser wenigstens 50 Gew.-%, noch besser wenigstens 75 Gew.-% und am besten die Gesamtmenge (insbesondere der unten genannten relativen Molekulargewichte)) von in der flüssigen Monomerenphase gelöst enthaltenem Polymerisat des Monomeren unterworfen wird, dass die wenigstens eine Trennoperation eine Filtration ist, bei der der Abscheidegrad des eingesetzten Filtriermediums für Partikel mit einem Teilchendurchmesser ≥ 30 µm wenigstens 90 % beträgt und dass zur Einhaltung einer Lagertemperatur eine Teilmenge der flüssigen Monomerenphase entnommen, über einen Wärmetauscher geführt und in den Lagerbehälter zurückgeführt wird.

Der Begriff Polymerisat soll hier auch Oligomerisat umfassen. In erster Linie ist darunter durch (unerwünschte) radikalische Polymerisation erzeugtes Polymerisat/Oligomerisat gemeint.

Der Wortlaut "in der flüssigen Monomerenphase gelöst enthaltenem Polymerisat" soll in dieser Schrift sowohl "molekular gelöst" als auch "kofloidaf gelöst" umfassen. Beides aber nur insoweit, als die gelöste Form in der zu lagernden flüssigen Monomerenphase visuell (d.h., mit bloßem menschlichem Auge) nicht wahrnehmbar ist.

Eigene Untersuchungen haben ergeben, dass insbesondere derartiges gelöstes Polymerisat (im Unterschied zu makroskopisch sichtbarem Polymerisat) eine ausgeprägt polymeris.ationsfördernde Aktivität aufweist. Dies gilt vor allem bei auf atomaren Wasserstoff bezogenen relativen Molekulargewichten von ≥ 1000, bzw ≥ 2000 bzw. ≥ 3000 des gelösten Polymerisats.

Die unerwünschte Bildung von derartigem Polymerisat kann selbst bei einer Lagerung der flüssigen Monomerenphase bei tiefen Temperaturen (≤ 50 °C, besser ≤ 40 °C, oder ≤ 30 °C, jedoch oberhalb des Festpunktes der Monomerenphase) nicht völlig verhindert werden. Bevorzugte Lagertemperaturen betragen 17 bis 25°C. Detaillierte Untersuchungen haben zum Ergebnis geführt, dass sich solchermaßen in entnommener flüssiger Monomerenphase gelöst enthaltenes Polymerisat bei Verwendung von nicht einem erfindungsgemäßen Verfahren unterzogener entnommener flüssiger Monomerenphase für radikalische Polymerisationen sowohl auf den Verlauf der radikalischen Polymerisation als auch auf die Qualität des resultierenden Polymerisats nachteilig auswirkt.

Bei einem Verfahren der z. B. radikalischen Polymerisation wird daher erfindungsgemäß transportierte flüssige Monomerenphase oder deren Gemisch mit von der transportierten flüssigen Monomerenphase verschiedenen wenigstens einfach ungesättigten (vorzugsweise ethylenisch) Verbindungen einpolymerisiert (z. B. radikalisch initiiert).

D. h., das erfindungsgemäße Verfahren ist insbesondere auch auf aus einem Lagerbehälter entnommene flüssige Monomerenphasen anwendbar, die bei visueller Betrachtung (d. h., bei Betrachtung mit bloßem menschlichem Auge) frei von Feststoffen sind.

Als Trennoperation zur Abtrennung von in der flüssigen Monomerenphase gelöst enthaltenem Polymerisat können insbesondere alle mechanischen Trennoperationen dienen, die zur Abtrennung von feinstteiligen Feststoffen von Flüssigkeiten geeignet sind. Diesbezüglich zu nennen sind filtrierende und/oder zentrifugierende Trennoperationen. Dazu zählen im besonderen die Mikrofiltration, die Ultrafiltration (Nanofiltration) und das Ultrazentrifugieren (die Massendichten von oligomerisiertem und/oder polymerisiertem Monomer sind von der Massendichte des entsprechenden Monomeren ebenso ausreichend verschieden wie die jeweiligen Volumenausdehnungen; bei 25°C und 1 atm beträgt die Massendichte von monomerer Acryisäure beispielsweise 1,05 g/cm³ und von polymerisierter Acrylsäure 1,54 g/cm³; der Unterschied in der Massendichte fußt insbesondere auf dem erhöhten Raumbedarf der Elektronenwolke einer ungesättigten Doppelbindung; die oligo- und/oder polymerisierten Monomeren reichern sich am Mantel der Ultrazentrifuge an und können von selbigem kontinuierlich abgeschält werden. Selbstverständlich können auch chromatographische sowie osmotische Verfahren als Trennoperation angewendet werden.

Erfindungsgemäß bevorzugt wird man das in der entnommenen (bzw. zu entnehmenden) flüssigen Monomerenphase (visuell nicht wahrnehmbar) gelöst enthaltene Polymerisat durch Filtration von der flüssigen Monomerenphase abtrennen. Als Filtermaterialien können dabei z.B. Filtervliese, Filtergewebe, Faserschichten, Sintermaterialien oder Schüttschichten (z.B. aus feinteiligen Quarzmaterialien, Kieselgur, Aktivkohle, Zeolithe) verwendet werden.

Grundsätzlich können für den erfindungsgemäßen Anwendungszweck z.B. Beutelfilter oder Kerzenfilter eingesetzt werden. Die erfindungsgemäße Filtrationsaufgabe kann dabei sowohl mit genähten als auch mit voll verschweißten, vorzugsweise mehrlagigen Filterbeuteln aus unterschiedlichen Materialien gelöst werden. Als solche Materialien kommen z.B. Edelstahl, Polypropylen, Cellulose, Polyester, Metallgewebe (Edelstahl wie z.B. Chrom-Nickel-Edelstahl) sowie phenolharzgebundene Acrylfasern in Betracht. Erfindungsgemäß besonders bevorzugtes Material für erfindungsgemäß zu verwendende Filter (sowohl Filterbeutel als auch Filterkerzen) ist Polypropylen. Erfindungsgemäß verwendbare Filterkerzen können jedoch z.B. auch aus Aktivkohle gefertigt sein. Grundsätzlich kommen als erfindungsgemäß geeignete Filterkerzen sowohl Wickelkerzen, Meltblown-Kerzen und harzgebundene Filterkerzen in Betracht. Sowohl die Filterbeutel als auch die Filterkerzen können erfindungsgemäß sowohl in Einfach- und in Mehrfach-Filtergehäusen zur Anwendung kommen. Als Gehäusematerial kommt z.B. Polypropylen, Edelstahl und C-Stahl in Betracht. Erfindungsgemäß bevorzugt ist die Anwendung von Mehrfach-Gehäusen, in denen bis zu ca. 40 einzelne Filterbeutel eingesetzt werden können und die Durchflussmengen an flüssiger Monomerenphase von bis zu 1000 m³/h ermöglichen. Einbauhilfen oder Beutelniederhalter verbessern den korrekten Sitz der Filterbeutel. Beispielsweise verhindern sie sicher ein "Aufschwimmen" durch unkontrollierten Rückstau. Ebenso wird ein Platzen des Filterbeutels verhindert. Spezifische "Kragen" sorgen für perfekten und festen Sitz des Beutelniederhalters im Filterbeutel (kein Durchscheuern oder Erosion des Filterbeutels).

Erfindungsgemäß werden Filtermaterialien (Filtermedien bzw. Filtertypen) eingesetzt, deren Abscheidegrad (retention efficiency) für Partikel mit einem Teilchendurchmesser von ≥ 30 µm wenigstens 90 % bzw. wenigstens 95 % und mehr beträgt.

Besonders bevorzugt werden beim erfindungsgemäßen Verfahren Filtermaterialien (Filtermedien bzw. Filtertypen) eingesetzt, deren Abscheidegrad für Partikel mit einem Teilchendurchmesser von ≥ 20 µm wenigstens 70 %, vorzugsweise wenigstens 80 %, besonders bevorzugt wenigstens 90 % bzw. wenigstens 95 % und mehr beträgt.

Ganz besonders bevorzugt werden beim erfindungsgemäßen Verfahren Filtermaterialien (Filtermedien bzw. Filtertypen) eingesetzt, deren Abscheidegrad für Partikel mit einem Teilchendurchmesser von ≥ 10 µm wenigstens 70 %, vorzugsweise wenigstens 80 %, besonders bevorzugt wenigstens 90 % bzw. wenigstens 95 % und mehr beträgt.

Noch besser werden beim erfindungsgemäßen Verfahren Filtermaterialien (Filtermedien bzw. Filtertypen) eingesetzt, deren Abscheidegrad für Partikel mit einem Teilchendurchmesser von ≥ 5 µm wenigstens 70 %, vorzugsweise wenigstens 80 %, besonders bevorzugt wenigstens 90 % bzw. wenigstens 95 % und mehr beträgt.

Am besten werden beim erfindungsgemäßen Verfahren Filtermaterialien (Filtermedien bzw. Filtertypen) eingesetzt, deren Abscheidegrad für Partikel mit einem Teilchendurchmesser von ≥ 1 µm wenigstens 70 %, vorzugsweise wenigstens 80 %, besonders bevorzugt wenigstens 90 % bzw. wenigstens 95 % und mehr beträgt.

In der Regel werden für das erfindungsgemäße Verfahren (sowie für das später in dieser Schrift noch ausgeführte Entnahmeverfahren) jedoch Filtermaterialien (Filtermedien bzw. Filtertypen) eingesetzt, deren Abscheidegrad für Partikel mit einem Teilchendurchmesser von 0,5 µm bei ≤ 90 %, vorzugsweise bei ≤ 80 % liegt. Dadurch werden für das erfindungsgemäße Verfahren in der Regel befriedigende Raum-Zeit-Ausbeuten gewährleistet.

Die vorgenannten Prozentsätze beziehen sich jeweils auf die Gesamtpartikelanzahl der jeweiligen Partikelgröße. Ferner beziehen sich vorgenannte Prozentsätze auf eine Flächenbelastung von 20 l/(m^{2.}min), Wasser als Trägermedium für die Partikel, eine Temperatur von 20°C und eine Druckdifferenz < 50 mbar, sowie Teststaub gemäß ISO 12103-1 A3 als Partikelart.

Die Testausführung erfolgt in Anlehnung an den French standard NF 45-303 (vgl. Filtration & Separation (Filtr. sep.) ISSN 0015-1882 CODEN FSEPAA Filtration and Separation, 1997, vol. 34, n°3, pp. 217-223).

Alle vorgenannten Eigenschaften erfüllen beispielsweise ACCUGAF™ Filterbeutel der Firma Eaton Filtration, LLC., 900 Fairmount Avenue, Elizabeth, New Jersey 07207 (USA) der Filtermodelle AGF-51, AGF-53, AGF-55, AGF-57 und AGF-59. Sie sind aus schmelzgeblasenem Polypropylen in verschweißter Form gefertigt. Sie weisen keine Bindemittel, keine grenzflächenaktiven Substanzen und keine Additive (z.B. klebende Harze) auf. Derartige, nur aus Polypropylen gefertigte, Filtermaterialen (Filtermedien , Filtertypen) erweisen sich für das erfindungsgemäße Verfahren als besonders geeignet, da sie die erfindungsgemäß zu lagernden flüssigen Monomerphasen in besonders geringfügiger Weise zu unerwünschter radikalischer Polymerisation reizen.

Die höchsten Abscheidegrade werden dabei in Anwendung des Typs AGF-51 erreicht (wenigstens 90 % für Partikeldurchmesser ≥ 1 µm). Mit zunehmender numerischer Kennzahl nehmen die Abscheidegrade ab. Für den Typ AGF-59 liegt der Abscheidegrad für Parikeldurchmesser ≥ 30 µm jedoch noch immer oberhalb von 90 %.

Alternativ können für das erfindungsgemäße Verfahren auch Filterbeutel PROGAF™ oder LOFCLEAR™ der Firma Eaton verwendet werden. Bei den LOFCLEAR Filterbeuteln handelt es sich um Filterbeutel aus mehrlagigem Filtermaterial und bei den PRO-GAF™ Filterbeuteln handelt es sich um Filterbeutel aus besonders hoch effizientem Filtermedium, mit dem effektive Partikelreduktionen bis in den Submikron-Bereich erreicht werden können. LOFCLEAR Filterbeutel sind ebenfalls aus 100 % Polypropylen gefertigt.

Alternativ können für ein erfindungsgemäßes Verfahren die Hochleistungs-Beutelfilter der Baureihe "HP" (insbesondere die Beutelfilter HPC, HPB und HPA) der Pall GmbH, Philipp-Reis-Straße 6, D-63303 Dreieich verwendet werden. Sie bestehen aus drei übereinander angeordneten Polypropylen-Vliesen; einem äußeren Stützvlies, das eine gute mechanische Festigkeit des Beutelfilters gewährleistet, dem mittleren aktiven Filtervlies und einem inneren Schutzvlies, das als Vorfilter fungiert.

Günstig sind Beutelfilter, deren Differenzdruck im Neuzustand bei einer Belastung mit 10 (vorzugsweise mit 20) m³/(h • m²) 20°C aufweisendem Wasser ≤ 100 mbar beträgt. In der Regel liegt der vorgenannte Wert jedoch bei ≥ 5 mbar.

Als erfindungsgemäß geeignet seien weiterhin aus Polypropylen gefertigte Kerzenfilter der Firma FUHR GmbH (D-55270 Klein-Winternheim bei Mainz, Am Weinkastell 14) erwähnt. Dazu zählen insbesondere die acuraProgard Membranfilterkerzen, die ebenfalls vollständig aus Polypropylen gefertigt sind. Sie weisen eine thermische Schweißkonstruktion auf und sind dadurch frei von Kleber und Bindemitteln. Da ihre Filtermatrix eine zweilagige Struktur aufweist, können bei ihrer Verwendung äußerst lange Standzeiten erreicht werden.

acuraPrograd Kerzenfilter sind mit Abscheideraten von wenigstens 99,9 % für Partikeldurchmesser ≥ 0,2 µm, bzw. ≥ 0,45 µm, bzw. ≥ 1 µm, bzw. ≥ 5 µm, bzw. ≥ 10 µm oder ≥ 20 µm erhältlich. Sie sind alle für das erfindungsgemäße Verfahren geeignet. Beispielhaft hervorgehoben sei die Verwendung der acuraPrograd Kerzenfilter mit Abscheideraten von mindestens 99,9 % für Partikeldurchmesser ≥ 5 µm.

Die Temperatur der erfindungsgemäß zu filtrierenden Monomerenphase sollte insbesondere bei Verwendung von Filtermedien aus Polypropylen ≤ 95°C betragen.

Vorzugsweise liegt vorgenannte Temperatur bei ≤ 80°C, besonders bevorzugt bei ≤ 60°C, ganz besonders bevorzugt bei ≤ 40°C und am besten bei ≤ 30 °C bzw. ≤ 25°C. In günstigen Fällen kann sie bis zu 0 °C betragen.

Im übrigen wird die wenigstens eine Trennoperation des erfindungsgemäßen Verfahrens generell vorzugsweise bei Temperaturen ≤ 95°C, bevorzugt ≤ 80°C, vorteilhaft ≤ 60°C, besonders vorteilhaft ≤ 40°C und am besten bei ≤ 30 °C bzw. ≤ 25°C durchgeführt. In günstigen Fällen kann sie bis zu 0 °C betragen. Die Transporttemperaturen sind gleichfalls vorzugsweise gering (vorzugsweise ≤ 30 °C, typisch 17 bis 25°C).

Bei einer wie beschrieben erfindungsgemäß durchzuführenden Filtration können Differenzdrucke von ≥ 10 mbar bis 5 bar angewendet werden.

Vorzugsweise liegen vorgenannte Differenzdrucke bei ≥ 20 mbar und ≤ 3 bar, besonders bevorzugt bei ≥ 20 mbar und ≤ 2 bar, bzw. ≤ 1,5 bar. Werden höhere Differenzdrucke erforderlich, sollten die Filter ausgetauscht werden. Selbstverständlich kommen für eine erfindungsgemäße Filtration aber auch Filterkerzen aus Polytetrafluorethylen (z. B. die acuraVent AVF Filterkerzen der FUHR GmbH), aus Borasilikatfasern (z. B. die acuraVent AFG-GG Filterkerzen der FUHR GmbH), aus Polyethersulfon (z. B. die acuraFine AFS Filterkerzen der FUHR GmbH), sowie aus Nylon (z. B. die acuraPrograd Filterkerzen der FUHR GmbH) in Betracht.

Selbstverständlich können für eine erfindungsgemäße Filtration auch die acuraBag Filterbeutel der FUHR GmbH, insbesondere diejenigen, die aus Polypropylen gefertigt sind, verwendet werden. Erfindungsgemäß geeignet sind aber auch die ACCUFIT® und die ULTRAFIT®-Filterbeutel der FUHR GmbH. Sie sind erfindungsgemäß vorteilhaft ebenfalls aus Polypropylen in verschweißter Ausführung gefertigt.

In der Regel sind erfindungsgemäß geeignete Filterbeutel aus mehreren Filterlagen gefertigt.

Erfindungsgemäß vorteilhaft ist, wenn sich das für ein erfindungsgemäß eingesetztes Verfahren eingesetzte Filtermedium unter der Belastung mit zu filtrierender flüssiger Monomerenphase nicht wesentlich ausdehnt. Vorgenannte Belastung wird in der Regel 2 bis 20 m³/(h• m²) betragen.

In der Regel wird man die zu filtrierende flüssige Monomerenphase bei Verwendung von Filterbeuteln oder Filterkerzen von innen nach außen durch den Filtertyp führen.

Grundsätzlich kann bei Verwendung von Filterkerzen die Führung der flüssigen Monomerenphase auch von außen nach innen erfolgen.

Typische Porengrößen der filteraktiven Schicht von erfindungsgemäß zu verwendenden Filtermaterialien betragen 0,1 bis 300 µm, vorzugsweise 1 bis 200 µm bzw. 1 bis 100 µm und besonders bevorzugt 1 bis 50 µm bzw. 1 bis 5 µm. Wie bereits erwähnt, werden erfindungsgemäß vorteilhaft mehrere Lagen (z. B. 3, oder 5, oder 7 Lagen) übereinander angewendet.

Die Filtration kann grundsätzlich als Druck- oder als Vakuumfiltration praktiziert werden. Vorzugsweise wird sie als Druckfiltration durchgeführt. Selbstverständlich kann sie auch zentrifugierend praktiziert werden.

Das erfindungsgemäße Verfahren eignet sich insbesondere auch dann, wenn der Gehalt der flüssigen Monomerenphase an dem Monomeren ≥ 96 Gew.-%, oder ≥ 97 Gew.-%, oder ≥ 98 Gew.-%, oder ≥ 99 Gew.-%, oder ≥ 99,5 Gew.-%, oder ≥ 99,7 Gew.-%, oder ≥ 99,9 Gew.-% beträgt. Das Vorgenannte und alle Aussagen in dieser Schrift sind insbesondere dann zutreffend, wenn das Monomere Acrylsäure ist (insbesondere wenn die flüssige Monomerenphase eine Acrylsäure ist (insbesondere wenn die flüssige Monomerenphase eine Reinacrylsäure ist).

Ist ein erfindungsgemäß verwendeter Filtertyp erschöpft, kann er durch einen frischen Filter ersetzt werden. Selbstredend kann der Filter zielgerichtet als Wechselfilter gestaltet werden. Durch Waschen mittels wässrigem Alkalihydroxid (z. B. Kaliumhydroxid und/oder Natriumhydroxid) und nachfolgendes Neutralwaschen mit reinem Wasser können erfindungsgemäß erschöpfte Filter regeneriert werden. Insbesondere aus Polypropylen gefertigte Filter können nach ihrer Erschöpfung problemlos verbrannt werden.

Als eine unerwünschte radikalische Polymerisation des in der flüssigen Monomerenphase befindlichen Monomeren (z. B. ausgelöst durch Temperatur, Licht oder sonstige spontan ausgelöste Radikalbildung) inhibierende Wirkstoffe kann die erfindungsgemäß entnommene flüssige Monomerenphase jeden der im Stand der Technik für diesen Zweck bekannten Polymerisationsinhibitoren gelöst enthalten. Bevorzugt verwendete Polymerisationsinhibitoren sind p-Methoxyphenol (MEHQ), Phenothiazin, Hydrochinone, Phenol (z. B. 2,4-Dimethyl-6,6-butylphenol), Chinone, Butylbrenzkatechin, Diphenylamin, p-Phenylendiamine, Nitroxyl-Radikale (z. B. 4-Hydroxy-2,2,6,6-tetramethylpiperidin-1-oxyl(4-OH-THEMPO)) und/oder Nitrosoverbindungen wie z. B. Nitrophenole (sowie alle anderen in der WO 00/64947 genannten Polymerisationsinhibitoren).

Die erfindungsgemäße Verfahrensweise ermöglicht es, den Inhibitorgehalt der zu transportierenden flüssigen Monomerenphase bei sicherem Transport ebenso zu verringern wie den Sauerstoffgehalt in der über der flüssigen Phase im Transporttank befindlichen Gasphase.

Selbstredend hängt die erforderliche Menge des verwendeten Inhibitors auch von den Lagerbedingungen, von der Art des Inhibitors, von der Art des Monomeren und von der spezifischen Reinheit der flüssigen Monomerenphase ab. Bezogen auf die in der flüssigen Monomerenphase enthaltene Gewichtsmenge an Monomerem beträgt der Inhibitorgehalt typisch ≤ 400 Gew.-ppm, häufig ≤ 220 Gew.-ppm und oft ≤ 100 Gew.-ppm.

Zweckmäßigerweise wird man bei der erfindungsgemäßen Befüllung eines Tankwagens oder Tankschiffs aus einem die flüssige Monomerenphase enthaltenden Lagerbehälters heraus so vorgehen, dass man die zu entnehmende flüssige Monomerenphase aus dem Lagertank zunächst als solche in einen Prüftank hinein entnimmt (selbstredend könnte die erfindungsgemäße Abtrennung bereits unmittelbar bei der Entnahme erfolgen (z. B. durch ein in der Entnahmeleitung bzw. an der Entnahmestelle im Lagerbehälter befindliches Filter).

Aus dem Prüftank wird die dem Lagertank entnommene flüssige Monomerenphase dann ein- oder mehrfach aufeinanderfolgend herausgepumpt und z. B. über ein wie vorstehend beschriebenes Filtersystem in den Prüftank jeweils wieder rückgeführt.

Nimmt die Abscheidemenge im Filtersystem (z. B. acuraPrograd Kerzenfilter mit Abscheideraten von 99,9 % für Partikeldurchmesser ≥ 5 um in einem Mehrfach-Gehäuse) nicht mehr oder nicht mehr nennenswert zu, wird der Trennvorgang abgebrochen und die im Prüftank befindliche, dem Lagertank zuvor entnommene Monomerenphase in den Tank des Tankwagens oder des Tankschiffs überführt.

Die vorliegende Anmeldung umfasst daher auch ein Verfahren zum Befüllen des Tanks eines Tankwagens oder eines Tankschiffs mit einem Lagertank zu entnehmender flüssiger Monomerenphase, wie in Anspruch 1 definiert.

Die Lagerung der flüssigen Monomerenphase im Lagerbehälter kann im übrigen z. B. so erfolgen, wie es die WO 2005/049543, die US 6,910,511, Research Disclosure Database Number 513002, Published in January 2007 und insbesondere Research Disclosure Database Number 513001, Published in January 2007, beschreiben.

Erfolgt die Erzeugung der zu lagernden flüssigen Monomerenphase z. B. in einer Rektifikationskolonne durch Kondensation oberhalb der Zufuhrstelle (Zulaufstelle) des (in der Rektifikationskolonne) aufzutrennenden Gemischs in die Rektifikationskolonne aus den in der Rektifikationskolonne aufsteigenden Dämpfen heraus, und wird die so kondensativ erzeugte und erfindungsgemäß zu lagernde flüssige Monomerenphase dann oberhalb des vorgenannten Zulaufs aus der Rektifikationskolonne entnommen, kann die Kondensation innerhalb der Rektifikationskolonne z. B. durch direkte Kühlung und/oder indirekte Kühlung bewirkt werden. Eine indirekte Kühlung kann z. B. am Kolonnenkopf dadurch bewirkt werden, dass man den am Kolonnenkopf ankommenden aufgestiegenen Dampf durch einen indirekten Wärmeaustauscher führt und die unter den Bedingungen des indirekten Wärmeaustauschs kondensierbaren Bestandteile in die flüssige Phase überführt. Ein Teil der so erzeugten flüssigen Phase wird dann der Lagerung zugeführt und der andere Teil als Rücklaufflüssigkeit auf den Kopf der Rektifikationskolonne rückgeführt. Diese Rücklaufflüssigkeit bedingt dann in der Rektifikationskolonne bereits eine direkte Kühlung des in der Rektifikationskolonne aufsteigenden Dampfes. Im indirekten Wärmeaustauscher nicht kondensierbare Bestandteile werden aus der Rektifikationskolonne heraus- und in der Regel ihrer Entsorgung zugeführt. Der Polymerisationsinhibitor wird unmittelbar ins Kondensat zugegeben. Erfolgt die Reinproduktentnahme unterhalb des Kolonnenkopfes, wird die am Kopf kondensierte Phase normalerweise weitgehend als Rücklaufflüssigkeit in die Rektifikationskolonne rückgeführt. Üblicherweise enthält die Rücklaufflüssigkeit Polymerisationsinhibitor zugesetzt. Das aus der Rektifikationskolonne entnommene Reinprodukt ist so unmittelbar ebenfalls polymerisationsinhibiert. Selbstredend kann die Kondensation am Kolonnenkopf auch ausschließlich durch direkte Kühlung vorgenommen werden. Zu diesem Zweck wird einmal erzeugtes Kopfkondensat mit Inhibitor versetzt, abgekühlt und wenigstens teilweise in den Kopfraum der Rektifikationskolonne zum Zweck der Direktkühlung versprüht. Erfolgt die Reinproduktentnahme unterhalb des Kolonnenkopfes (aber oberhalb der Zufuhrstelle des aufzutrennenden Gemischs in die Rektifikationskolonne) wird normalerweise die abgekühlte Kondensatgesamtmenge in den Kopfraum rückversprüht. Der trennwirksame Einbauten enthaltende Teil der Rektifikationskolonne und der Kondensationsraum am Kolonnenkopf sind üblicherweise durch einen Kaminboden voneinander getrennt. Die Rücklaufflüssigkeit wird dem trennwirksamen Teil zugeführt. Der unterhalb der Zufuhrstelle (Zulaufstelle) in die Rektifikationskolonne befindliche Teil der Rektifikationskolonne wird üblicherweise als Abtriebsteil und der oberhalb der Zufuhrstelle befindliche Teil der Rektifikationskolonne wird üblicherweise als Verstärkerteil der Rektifikationskolonne bezeichnet.

Erfolgt die Zufuhr des in der Rektifikationskolonne aufzutrennenden Stoffgemischs in die Rektifikationskolonne in flüssiger Form, bildet die Auftrennung eine Rektifikation, erfolgt die Zufuhr dampfförmig (bzw. gasförmig), bildet die Auftrennung eine fraktionierende Kondensation. Als trennwirksame Einbauten kann die Rektifikationskolonne z. B. Böden, Packungen und/oder Füllkörper enthalten. Beispielhafte Ausführungsformen einer fraktionierenden Kondensation von Acrylsäure aus dem Produktgasgemisch einer heterogen katalysierten partiellen Gasphasenoxidation von Propylen und/oder Propan offenbaren z. B. die Schriften DE-A 199 24 533, DE-A 199 24 532, WO 01/77056, DE-A 101 56 016, DE-A 102 43 625, DE-A 102 23 058, DE-A 102 35 847, WO 2004/035514, WO 00/53560 und DE-A 103 32 758.

Wird die zu lagernde Monomerenphase z. B. durch Aufschmelzen einer kristallinen Phase erzeugt, kann diese das Ergebnis einer einstufigen oder einer mehrstufigen kristallisativen Reinigung eines das relevante Monomere enthaltenden flüssigen Gemisches sein (vgl. z. B. EP-A 616 998). Durch Einwirkung von Kälte auf das flüssige Gemisch kristallisiert das Monomere aus dem flüssigen Gemisch als kristalline Phase aus.

Häufig ist die aufzuschmelzende kristalline Phase das Ergebnis einer einstufigen Kristallisation.

Selbstverständlich kann die aufzuschmelzende Phase auch das Ergebnis einer fraktionierten Kristallisation sein.

Beispielsweise kann die Kälteeinwirkung auf das das Monomere enthaltende flüssige Gemisch als Schichtkristallisation ausgeführt sein (vgl. DE-OS 26 06 364, EP-A 616 998, EP-A 648 520 und EP-A 776 875). Dabei wird das Kristallisat in Form zusammenhängender, fest anhaftender Schichten an gekühlten Oberflächen ausgefroren. Die Trennung des abgeschiedenen Kristallisats von der verbliebenen Restschmelze (der Mutterlauge) erfolgt durch einfaches Abfließen der Restschmelze. Prinzipiell unterscheidet man zwischen "statischen" und "dynamischen" Schichtkristallisationsverfahren. Kennzeichnend für die dynamische Schichtkristallisation aus flüssigen Gemischen ist eine erzwungene Konvektion des flüssigen Gemischs. Diese kann z. B. durch Umpumpen des flüssigen Gemischs durch gekühlte volldruchströmte Rohre, durch Aufgabe des flüssigen Gemischs als Rieselfilm auf gekühlte Wandungen (z. B. gemäß EP-A 616 998, z. B. in gekühlten Fallrohen) oder durch Einleiten von Inertgas in das flüssige Gemisch oder durch Pulsieren erfolgen.

Bei den statischen Verfahren ruht das flüssige Gemisch (z. B. in Rohrbündel oder Plattenwärmeaustauschern) und scheidet sich schichtförmig durch langsame Temperatursenkung auf der Sekundärseite des Wärmeaustauschers ab. Danach wird die Restschmelze (Mutterlauge) abgelassen, durch langsame Temperaturerhöhung stärker verunreinigte Fraktionen aus der Kristallschicht abgeschwitzt und nachfolgend das Reinprodukt abgeschmolzen (vgl. WO 01/77056).

Generell enthält das das Monomere enthaltende flüssige Gemisch (normalerweise eine Lösung), aus dem das Monomere kristallisativ abgetrennt wird, Polymerisationsinhibitor zugesetzt. Bei der kristallisativen Abscheidung des Monomeren kristallisiert dieses normalerweise im wesentlichen frei vom Polymerisationsinhibitor aus. Das Aufschmelzen der abgeschiedenen Monomerenkristallschicht erfolgt daher häufig nicht durch Erwärmen der Sekundärseite, sondern durch in Kontakt bringen der Monomerenkristallschicht mit einer Polymerisationsinhibitor zugesetzt enthaltenden erwärmten Schmelze von zuvor aufgeschmolzener abgeschiedener Monomerenkristallschicht.

Alternativ kann die Ausbildung des Monomerenkristallisats aus dem das Monomere enthaltenden flüssigen Gemisch auch als Suspensionskristallisation (z. B. gemäß der Lehre der WO 01/77056, der WO 02/055469 sowie der WO 03/078378) erfolgen.

Dabei wird in der Regel durch Kühlung des z. B. Acrylsäure als Monomeres enthaltenden flüssigen Gemischs (der flüssigen Lösung) eine Acrylsäurekristalle suspendiert enhaltende Kristallsuspensionen erzeugt, wobei die Acrylsäurekristalle einen geringeren und die verbliebene Restschmelze (Mutterlauge) einen höheren Verunreinigungsgehalt aufweist als das flüssige Ausgangsgemisch. Dabei können die Acrylsäurekristalle unmittelbar in Suspension befindlich wachsen und/oder sich als Schicht auf einer gekühlten Wand abscheiden, von der sie stetig abgekratzt und in der Restschmelze resuspendiert werden.

Alle in der WO 01/77056, der WO 02/055469 sowie in der WO 03/078378 ausgeführten Suspensionskristaller und Suspensionskristallisationsverfahren können dabei angewendet werden. Eine so erzeugte Acrylsäurekristallisatsuspension kann z. B. einen Feststoffgehalt von 10 bis 50, häufig 20 bis 40 oder 30 Gew.-% aufweisen.

Zur Trennung von Suspensionskristallisat und verbliebener Mutterlauge kommen z. B. alle in den vorgenannten WO-Veröffentlichungen genannten Trennverfahren in Betracht (z. B. mechanische Trennverfahren wie Zentrifugieren). Bevorzugt erfolgt die Trennung in einer Waschkolonne. Mit Vorteil handelt es sich dabei um eine Waschkolonne mit erzwungenem Transport der abgeschiedenen z. B. Acrylsäurekristalle. Als Waschflüssigkeit wird mit Vorteil die Schmelze von in der Waschkolonne vorab gereinigten (abgetrennten) z. B. Acrylsäurekristallen verwendet. Die Wäsche erfolgt normalerweise im Gegenstrom.

Alles Vorgenannte gilt vor allem dann, wenn die Waschkolonne eine Waschkolonne mit erzwungenem Transport der z. B. Acrylsäurekristalle ist, und dies vor allem dann, wenn es eine hydraulische oder eine mechanische Waschkolonne gemäß der WO 01/77056 ist und sie wie dort ausgeführt betrieben wird.

Eine zu lagernde flüssige Acrylsäurephase kann damit z. B. wie folgt erzeugt werden. Durch heterogen katalysierte einstufige oder zweistufige Partialoxidation eines C₃-Vorläufers der Acrylsäure (z. B. Propylen, Propan oder Acrolein) wird ein Acrylsäure enthaltendes Produktgasgemisch erzeugt. Dieses wird in eine Trennkolonne mit trennwirksamen Einbauten geführt und in selbiger aufsteigend fraktionierend kondensiert. Oberhalb der Zufuhrstelle aber unterhalb des Kolonnenkopfes wird aus der Trennkolonne eine flüssige rohe Acrylsäure entnommen, die ≥ 95 Gew.-% an Acrylsäure aufweist und über den Rücklauf in der Trennkolonne Polymerisationsinhibitor zugesetzt enthält. Durch Suspensionskristallisation wird aus der rohen Acrylsäure Reinacrylsäuresuspensionskristallisat erzeugt. Dieses wird in einer Waschkolonne (vorzugsweise in einer hydraulischen Waschkolonne) unter Verwendung von Polymerisationsinhibitor zugesetzt enthaltender Reinkristallisatschmelze als Waschflüssigkeit von verbliebener Mutterlauge abgetrennt. Durch in Kontakt bringen von abgetrenntem Reinkristallisat mit der Polymerisationsinhibitor zugesetzt enthaltenden Schmelze von zuvor abgetrenntem Reinkristallisat wird das abgetrennte Reinkristallisat aufgeschmolzen und so eine zu lagernde flüssige Acrylsäurephase erzeugt (Reinheit in der Regel ≥ 99,5 Gew.-%).

Selbstverständlich kann das erfindungsgemäße Verfahren auch auf flüssige Monomerenphasen angewendet werden, die auf andere Art und Weise erzeugt wurden und deren Gehalt an dem Monomeren < 90 Gew.-% beträgt.

Das erfindungsgemäße Verfahren kann auch so durchgeführt werden, dass man dem Lagertank im Verlauf der Lagerung immer wieder (am besten kontinuierlich) flüssige Monomerenphase entnimmt und diese anschließend wieder in den Lagertank rückführt und auf diesem Weg der Entnahme und Rückführung wenigstens einer Trennoperation zur Abtrennung wenigstens einer Teilmenge von in der flüssigen Monomerenphase gelöst enthaltenem Polymerisat des Monomeren unterwirft und von der so gelagerten flüssigen Monomerenphase in den Tank eines Tankwagens oder eines Tankschiffs überführt. In diesem Fall führt der erfindungsgemäße Weg der zu transportierenden flüssigen Monomerenphase aus dem Lagerbehälter in den Tank über den Lagerbehälter. Selbstverständlich können auch alle (oder Teilmenge derselben) in dieser Schrift vorgestellten erfindungsgemäßen Varianten kombiniert angewendet werden.

### Beispiel und Vergleichsbeispiel

### a) Vergleichsbeispiel

Durch fraktionierende Kondensation des Produktgasgemischs einer heterogen katalysierten partiellen Propylenoxidation wurde wie in Beispiel 1 der WO 2004/035514 beschrieben eine Acrylsäure erzeugt, die 96,9 Gew.-% Acrylsäure enthielt und mit 0,018 Gew.-% MEHQ sowie 0,012 Gew.-% Phenothiazin und 0,0004 Gew.-% molekularem Sauerstoff polymerisationsinhibiert war. Sie wurde vom zweiten Fangboden der Kondensationskolonne oberhalb der Zufuhr des Produktgasgemischs in die Kondensationskolonne mit einer Temperatur von 100,6 °C und visuell frei von Feststoffen aus der Kondensationskolonne entnommen. Dann wurde sie auf 25 °C abgekühlt und zwei Liter unter Luft in einem ein Innenvolumen von drei Liter aufweisenden Glaskolben überführt. Anschließen wurde der Glaskolben verschlossen vier Monate bei 20 °C gelagert. Dann wurden 0,5 ml der gelagerten Acrylsäure als solche (immer noch visuell frei von Feststoffen) entnommen und unter Luftatmosphäre in eine 1,8 ml Glasampulle überführt. Anschließend wurde die Ampulle bei 120 °C im Umlufttrockenschrank drehend gelagert, um vollständige Durchmischung zu gewährleisten. Dann wurde die Zeit t bis zu vollständigen Polymerisation der Probe erfasst, t betrug 13 h 12 Minuten.

### b) Beispiel

Es wurde wie im Vergleichsbeispiel verfahren. Bei der Entnahme aus dem Glaskolben wurde die gesamte gelagerte Acrylsäuremenge durch einen ACCUGAF AGF-51 Filterbeutel geführt. Nach beendeter Filtration wiesen die Innenwände des Filterbeutels einen klebrigen Belag aus Polyacrylsäure auf. 0,5 ml der filtrierten Acrylsäure wurden mit 20 °C und unter Luftatmosphäre in eine 1,8 ml Glasampulle überführt. Anschließend wurde die Ampulle bei 120°C im Umlufttrockenschrank drehend gelagert, um vollständige Durchmischung zu gewährleisten. Dann wurde die Zeit t bis zur vollständigen Polymerisation der Probe erfasst, t betrug 20 h 25 Minuten.

Wie die beiden Versuche ausweisen, mindert die Entfernung von gelöst enthaltenem Polymerisat (gelöst enthaltener Polyacrylsäure) die Neigung der Acrylsäure zu unerwünschter radikalischer Polymerisation.

Im Hinblick auf die obengenannten Lehren sind zahlreiche Änderungen und Abweichungen von der vorliegenden Erfindung möglich.

## Patentansprüche

1. Verfahren zum Befüllen des Tanks eines Tankwagens oder eines Tankschiffs mit einem Lagerbehälter zu entnehmender flüssiger Monomerenphase oder des Transports einer aus einem Lagerbehälter entnommenen flüssigen Monomerenphase im Tank eines Tankwagens oder eines Tankschiffs,
wobei der Gehalt der flüssigen Monomerenphase an dem Monomeren ≥ 90 Gew.-% beträgt und das Monomere ein Monomeres aus der Gruppe bestehend aus Acrolein, Methacrolein, Acrylsäure, Methacrylsäure, Ester aus Acrylsäure und einem 1 bis 12 C-Atome aufweisenden Alkohol sowie Ester aus Methacrylsäure und einem 1 bis 12 C-Atome aufweisenden Alkohol ist, **dadurch gekennzeichnet, dass** die flüssige Monomerenphase auf dem Weg aus dem Lagerbehälter in den Tank des Tankwagens oder des Tankschiffs wenigstens einer Trennoperation zur Abtrennung wenigstens einer Teilmenge von in der flüssigen Monomerenphase gelöst enthaltenem Polymerisat des Monomeren unterworfen wird, dass die wenigstens eine Trennoperation eine Filtration ist, bei der der Abscheidegrad des eingesetzten Filtriermediums für Partikel mit einem Teilchendurchmesser ≥ 30 µm wenigstens 90 % beträgt und dass zur Einhaltung einer Lagertemperatur eine Teilmenge der flüssigen Monomerenphase entnommen, über einen Wärmetauscher geführt und in den Lagerbehälter zurückgeführt wird.

2. Verfahren nach Anspruch 1, **dadurch gekennzeichnet, dass** das von einer fluiden Phase einnehmbare Innenvolumen des Lagerbehälters 100 m³ bis 10000 m³ beträgt.

3. Verfahren nach einem der Ansprüche 1 oder 2, **dadurch gekennzeichnet, dass** der Lagerbehälter eine Vorrichtung aufweist, mit Hilfe derer die Teilmenge der gelagerten flüssigen Monomerenphase entnommen, über einen Wärmeaustauscher geführt und anschließend in den Lagerbehälter rückgeführt wird.

4. Verfahren nach einem der Ansprüche 1 bis 3, **dadurch gekennzeichnet, dass** die flüssige Monomerenphase im Lagerbehälter unter einer molekularen Sauerstoff enthaltenden Atmosphäre gelagert wird.

5. Verfahren nach einem der Ansprüche 1 bis 4, **dadurch gekennzeichnet, dass** die flüssige Monomerenphase p-lviethoxyphenol und/oder Phenothiazin gelöst enthält.

6. Verfahren nach einem der Ansprüche 1 bis 5, **dadurch gekennzeichnet, dass** das bei der Filtration verwendete Filtermedium aus Polypropylen gefertigt ist.

7. Verfahren nach einem der Ansprüche 1 bis 5, **dadurch gekennzeichnet, dass** das bei der Filtration verwendete Filtermedium aus Edelstahl gefertigt ist.

8. Verfahren nach einem der Ansprüche 1 bis 7, **dadurch gekennzeichnet, dass** bei der Filtration der Abscheidegrad des eingesetzten Filtriermediums für Partikel mit einem Teilchendurchmesser ≥ 10 µm wenigstens 90 % beträgt.

9. Verfahren nach einem der Ansprüche 1 bis 8, **dadurch gekennzeichnet, dass** die wenigstens eine Trennoperation bei einer Temperatur von ≤ 50°C durchgeführt wird.

10. Verfahren nach einem der Ansprüche 1 bis 9, **dadurch gekennzeichnet, dass** das von einem fluiden Medium einnehmbare Innenvolumen des Tanks des Tankwagens oder Tankschiffs ≥ 5m³ beträgt.

11. Verfahren nach einem der Ansprüche 1 bis 10, **dadurch gekennzeichnet, dass** die flüssige Monomerenphase eine Reinacrylsäure ist, deren Acrylsäuregehalt ≥ 99 Gew.-% beträgt.

## Claims

1. A process for filling the tank of a tank truck or of a tanker ship with liquid monomer phase which is to be withdrawn from a storage vessel, or for transporting a liquid monomer phase which has been withdrawn from a storage vessel in the tank of a tank truck or of a tanker ship,
where the content in the liquid monomer phase of the monomer is ≥ 90% by weight and the monomer is a monomer from the group consisting of acrolein, methacrolein, acrylic acid, methacrylic acid, esters of acrylic acid and an alcohol having from 1 to 12 carbon atoms and esters of methacrylic acid and an alcohol having from 1 to 12 carbon atoms, wherein the liquid monomer phase, on the route from the storage vessel into the tank of the tank truck or of the tanker ship, is subjected to at least one separating operation to remove at least a portion of polymer of the monomer present dissolved in the liquid monomer phase, the at least one separating operation is a filtration in which the degree of retention efficiency of the filter medium used for particles having a particle diameter of ≥ 30 µm is at least 90%, and, to maintain a storage temperature, a portion of the liquid monomer phase is withdrawn, conducted through a heat exchanger and recycled into the storage vessel.

2. The process according to claim 1, wherein the internal volume of the storage vessel occupiable by a fluid phase is from 100 m³ to 10 000 m³.

3. The process according to either of claims 1 and 2, wherein the storage vessel has a device with the aid of which a portion of the stored liquid monomer phase is withdrawn, conducted through a heat exchanger and then recycled into the storage vessel.

4. The process according to any of claims 1 to 3, wherein the liquid monomer phase in the storage vessel is stored under an atmosphere comprising molecular oxygen.

5. The process according to any of claims 1 to 4, wherein the liquid monomer phase comprises dissolved p-methoxyphenol and/or phenothiazine.

6. The process according to any of claims 1 to 5, wherein the filter medium used in the filtration has been manufactured from polypropylene.

7. The process according to any of claims 1 to 5, wherein the filter medium used in the filtration has been manufactured from stainless steel.

8. The process according to any of claims 1 to 7, wherein in the filtration the degree of retention efficiency of the filter medium used for particles having a particle diameter of ≥ 10 µm is at least 90%.

9. The process according to any of claims 1 to 8, wherein the at least one separating operation is performed at a temperature of ≤ 50°C.

10. The process according to any of claims 1 to 9, wherein the internal volume of the tank of the tank truck or tanker ship occupiable by a fluid medium is ≥ 5 m³_{.}

11. The process according to any of claims 1 to 10, wherein the liquid monomer phase is glacial acrylic acid whose acrylic acid content is ≥ 99% by weight.

## Revendications

1. Procédé de remplissage de la citerne d'un camion-citerne ou d'un navire-citerne avec une phase monomère liquide à prélever dans un contenant de stockage ou de transport d'une phase monomère liquide prélevée dans un contenant de stockage dans la citerne d'un camion-citerne ou d'un navire-citerne,
la teneur de la phase monomère liquide en le monomère étant ≥ 90 % en poids et le monomère étant un monomère du groupe constitué par l'acroléine, la méthacroléine, l'acide acrylique, l'acide méthacrylique, les esters de l'acide acrylique et d'un alcool comprenant 1 à 12 atomes C, ainsi que les esters de l'acide méthacrylique et d'un alcool comprenant 1 à 12 atomes C, **caractérisé en ce que** la phase monomère liquide est soumise à au moins une opération de séparation pour la séparation d'au moins une partie du polymère du monomère contenu sous forme dissoute dans la phase monomère liquide sur le trajet entre le contenant de stockage et la citerne du camion-citerne ou du navire-citerne, **en ce que** ladite au moins une opération de séparation est une filtration lors de laquelle le rendement du milieu de filtration utilisé pour les particules ayant un diamètre de particule 30 µm est d'au moins 90 % et **en ce que**, pour maintenir une température de stockage, une partie de la phase monomère liquide est soutirée, passée dans un échangeur de chaleur et recyclée dans le contenant de stockage.

2. Procédé selon la revendication 1, **caractérisé en ce que** le volume intérieur du contenant de stockage pouvant être occupé par une phase fluide est de 100 m³ à 10 000 m³_{.}

3. Procédé selon l'une quelconque des revendications 1 ou 2, **caractérisé en ce que** le contenant de stockage comprend un dispositif à l'aide duquel la partie de la phase monomère liquide stockée est soutirée, passée dans un échangeur de chaleur, puis recyclée dans le contenant de stockage.

4. Procédé selon l'une quelconque des revendications 1 à 3, **caractérisé en ce que** la phase monomère liquide est stockée dans le contenant de stockage dans une atmosphère contenant de l'oxygène moléculaire.

5. Procédé selon l'une quelconque des revendications 1 à 4, **caractérisé en ce que** la phase monomère liquide contient du p-méthoxyphénol et/ou de la phénothiazine sous forme dissoute.

6. Procédé selon l'une quelconque des revendications 1 à 5, **caractérisé en ce que** le milieu de filtration utilisé lors de la filtration est en polypropylène.

7. Procédé selon l'une quelconque des revendications 1 à 5, **caractérisé en ce que** le milieu de filtration utilisé lors de la filtration est en acier inoxydable.

8. Procédé selon l'une quelconque des revendications 1 à 7, **caractérisé en ce que** le rendement du milieu de filtration utilisé lors de la filtration pour les particules ayant un diamètre de particule 10 µm est d'au moins 90 %.

9. Procédé selon l'une quelconque des revendications 1 à 8, **caractérisé en ce que** ladite au moins une opération de séparation est réalisée à une température ≤ 50°C.

10. Procédé selon l'une quelconque des revendications 1 à 9, **caractérisé en ce que** le volume intérieur de la citerne du camion-citerne ou du navire citerne pouvant être occupé par un milieu fluide est ≥ 5m³_{.}

11. Procédé selon l'une quelconque des revendications 1 à 10, **caractérisé en ce que** la phase monomère liquide est un acide acrylique pur, dont la teneur en acide acrylique est ≥ 99 % en poids.
